(19) Europäisches Patentamt
European Patent Office
Office européen des brevets

(11) Veröffentlichungsnummer: **0 406 817 A1**

## (12) EUROPÄISCHE PATENTANMELDUNG

(21) Anmeldenummer: **90112722.5**

(22) Anmeldetag: **04.07.90**

(51) Int. Cl.5: **A61F  9/00,** G02B 27/10,
B23K 26/06

(30) Priorität: **04.07.89 DE 8908123 U**

(43) Veröffentlichungstag der Anmeldung:
**09.01.91 Patentblatt  91/02**

(84) Benannte Vertragsstaaten:
**AT CH DE FR GB IT LI SE**

(71) Anmelder: **Firma Carl Zeiss**

**D-7920 Heidenheim (Brenz)(DE)**
(84) **CH**

Anmelder: **CARL-ZEISS-STIFTUNG trading as CARL ZEISS**

**D-7920 Heidenheim (Brenz)(DE)**
(84) **GB**

(72) Erfinder: **Müller, Dieter**
**Finkenweg 21**
**D-7923 Königsbronn(DE)**
Erfinder: **Schwarz, Jürgen, Dr.**
**Am Haselstrauch 1**
**D-7082 Oberkochen(DE)**

(54) **Vorrichtung zur Bestimmung der Fokusebene unsichtbarer Therapie-Laserstrahlung in ophthalmologischen Geräten.**

(57) Für den Markierungsstrahl eines Laser-Therapie-gerätes wird eine Fokussierungsvorrichtung angegeben, die den Markierungsstrahl mit einfachen Mitteln (3,4) in vier Teilstrahlen (5a,5b,6a,6b) aufspaltet, welche im Fokus zu einem Lichtpunkt vereinigt werden.

# Fig.1

EP 0 406 817 A1

# VORRICHTUNG ZUR BESTIMMUNG DER FOKUSEBENE UNSICHTBARER THERAPIE-LASERSTRAHLUNG IN OPHTHALMOLOGISCHEN GERÄTEN

Die Neuerung betrifft eine Vorrichtung zur Bestimmung der Fokusebene unsichtbarer Therapie-Laserstrahlung in ophthalmologischen Geräten mit einem Beobachtungsstrahlengang für den sichtbaren Bereich des Spektrums, einer sichtbaren Markierungs-Laserstrahlung und Mitteln zur Strahlaufweitung der Laserstrahlung.

Ophthalmologische Laser-Therapiegeräte werden für Eingriffe im Bereich der vorderen und mittleren Augenmedien eingesetzt. Die Laserstrahlung wird dabei durch die Optik des ophthalmologischen Gerätes geführt und trifft auf das Patientenauge in der gleichen Ebene, in der der Beobachtungsstrahl fokussiert ist. Als Therapie-Laserstrahlquelle bzw. als Wirkungsstrahl-Quelle dient meist ein Nd-YAG-Laser, der im unsichtbaren Bereich des Spektrums emittiert. Zur Kontrolle der Fokusebene der Laser-Therapiestrahlung wird deshalb der unsichtbaren Therapiestrahlung eine sichtbare Markierungsstrahlung überlagert, die meist aus einem He-Ne-Laser stammt und deren Fokusebene mit der Fokusebene der Wirkungsstrahlung übereinstimmt. Zur Kontrolle der Fokusebene der Wirkungsstrahlung kann dann die Fokusebene der Markierungsebene bestimmt werden.

Aus der DE-OS 3 331 586 ist ein Mechanismus zur Bestimmung der Fokusebene bekannt, der darin besteht, daß zwei Sektorteilbündel des Markierungs-Lasers abwechselnd aus- und eingeschaltet werden. Der Benutzer hat den Eindruck des Blinkens, wenn der Strahlengang von einer Ebene geschnitten wird, die nicht mit der Fokusebene identisch ist. Diese Fokusierhilfe benötigt an zwei Stellen des Markierungsstrahles strahlaufweitende Mittel sowie Abblendstreifen zum Ausblenden von Segmenthälften aus der Markierungsstrahlung.

Der vorliegenden Neuerung liegt die Aufgabe zugrunde, eine gegenüber dem Stand der Technik vereinfachte Vorrichtung zur Bestimmung der Fokusebene eines medizinischen Wirkungslaserstrahls mittels eines sichtbaren Laserstrahles anzugeben. Diese Aufgabe wird neuerungsgemäß dadurch gelöst, daß zwischen der Markierungs-Laserquelle und den strahlaufweitenden Mitteln zwei planparallele Glasplatten vorgesehen sind, die einen Winkel von 90° zwischen sich einschließen und von denen jede z.B. um 45° zur optischen Achse der Vorrichtung geneigt ist.

Die mit der Neuerung erzielbaren Vorteile bestehen einmal in einer Kostenreduzierung des Fokussierungssystems, zum anderen in einer drastischen Verringerung der störenden Lichtreflexe aus Ebenen außerhalb der Fokusebene und in einer Erleichterung der Fokussierung am Patientenauge, insbesondere außerhalb der optischen Achse des Auges mit der dort stark auftretenden astigmatischen Abbildung.

Ein Ausführungsbeispiel der Neuerung ist in der Zeichnung dargestellt und wird im folgenden näher beschrieben. Es zeigen

Figur 1 eine schematische Darstellung des Strahlenverlaufes zwischen dem Markierungs-Laser und der Fokussierungsebene;

Figur 2 den Strahlenverlauf in einer planparallelen Platte.

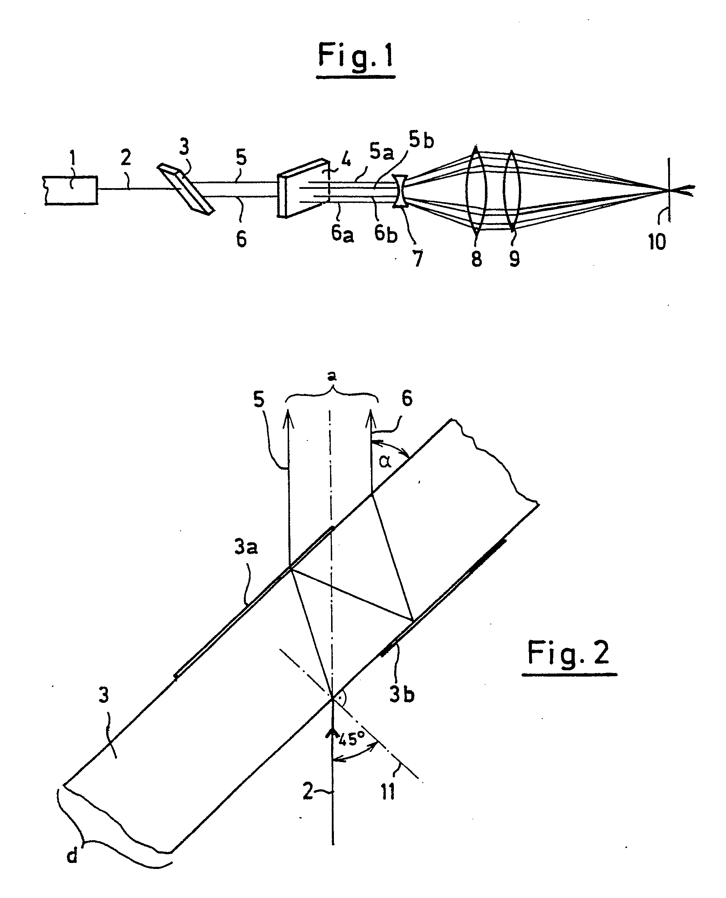

In der Darstellung der Figur 1 ist mit dem Bezugszeichen (1) ein He-Ne-Laser bezeichnet. Die optische Achse trägt die Bezeichnung (2). Mit dem Bezugszeichen (3) und (4) sind planparallelen Glasplatten bezeichnet, die so zur optischen Achse (2) geneigt sind, daß sie einen Winkel von 90° zwischen sich einschließen. Ein auf der optischen Achse (2) verlaufender Laserstrahl wird von der Glasplatte (3) in zwei Strahlen (5) und (6) aufgespalten. Beim Auftreffen dieser Strahlen (5) und (6) auf die Glasplatte (4) findet eine weitere Aufspaltung der Strahlen in die Strahlen (5a, 5b) und (6a, 6b) statt. Mit (7 und 8) ist eine Strahlaufweitungsoptik bezeichnet, die danach den Markierungs-Laserstrahl mit der Optik (9) in der Ebene (10) fokussiert, in der sich auch die Beobachtungsebene und die Behandlungsebene befindet.

In der Darstellung der Figur 2 ist die Aufspaltung eines unter dem Winkel von 45° zum Lot (11) auf die Glasplatte (3) auftreffenden Stahles (2) dargestellt. An der teilreflektierenden Schicht (3a) tritt ein Teil des Strahles (2) aus der Platte aus, der andere Teilstrahl wird zur reflektierenden Schicht (3b) gelenkt, von dort auf die gegenüberliegende Seite der Glasplatte (3), wo er unter dem Winkel α austritt. Der Abstand a der Teilstrahlen (5) und (6) hängt ab von der Dicke d und dem Brechungsindex n der Glasplatte sowie vom Winkel α. Durch entsprechende Dimensionierung ist es möglich, die vier aus der Glasplatte (4) austretenden Teilstrahlen symmetrisch zum eintretenden Strahl (2) verlaufen zu lassen. Die Planplatten (3) und (4) sind in einem Bereich von ± 0,5 mm unempfindlich gegenüber Verschiebungen die senkrecht zur optischen Achse verlaufen. Im Fokus ist ein Zielstrahldurchmesser von ca. 15 μm erreichbar. Bei Defokussierung sind entsprechend den vier Teilstrahlen vier Lichtpunkte zu erkennen, in der Fokusebene sind die vier Lichtpunkte zu einem Lichtpunkt verschmolzen.

**Ansprüche**

1. Vorrichtung zur Bestimmung der Fokusebene unsichtbarer Therapie-Laserstrahlung in ophthalmologischen Geräten mit einem Beobachtungsstrahlengang für den sichtbaren Bereich des Spektrums, einer sichtbarer Markierungs-Laserstrahlung und Mitteln zur Strahlenaufweitung der Laserstrahlung, dadurch gekennzeichnet, daß zwischen der Markierungs-Laserquelle und den strahlenaufweitenden Mitteln zwei planparallele Glasplatten vorgesehen sind, die einen Winkel von 90° zwischen sich einschließen und von denen jede z. B. um 45° zur optischen Achse geneigt ist.

# Fig.1

# Fig.2

Europäisches
Patentamt

**EUROPÄISCHER RECHERCHENBERICHT**

Nummer der Anmeldung

EP 90 11 2722

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | GB-A-2 149 142 (COOPER) * Zusammenfassung; Seite 2, Zeilen 2-42; Figuren 3,4 * | 1 | A 61 F 9/00 G 02 B 27/10 B 23 K 26/06 |
| Y | US-A-4 611 115 (RICH) * Spalte 3, Zeilen 4-22; Figur 3 * | 1 | |
| X,P | DE-U-8 908 123 (CARL ZEISS) * Insgesamt * | 1 | |
| A | GB-A-2 141 225 (CARL ZEISS) * Seite 1, Zeile 122 - Seite 2, Zeile 7; Figur 1 * | 1 | |
| A | US-A-3 794 407 (NISHIMURA) * Zusammenfassung; Figuren * | 1 | |
| A | US-A-4 541 688 (WATT) * Spalte 3, Zeile 54 - Spalte 4, Zeile 7; Figuren 2,3 * | 1 | |
| A | EP-A-0 293 126 (SCIENTIFIC GENERICS) | | **RECHERCHIERTE SACHGEBIETE (Int. Cl.5)** |
| A | US-A-4 830 483 (KOHAYAKAWA) | | A 61 F G 02 B B 23 K |
| A,D | EP-A-0 143 185 (CARL ZEISS) | | |

Der vorliegende Recherchenbericht wurde für alle Patentansprüche erstellt

| Recherchenort | Abschlußdatum der Recherche | Prüfer |
|---|---|---|
| DEN HAAG | 03-10-1990 | KLEIN C. |